(19) Europäisches Patentamt / European Patent Office / Office européen des brevets

(11) **EP 3 143 976 A1**

(12) **EUROPEAN PATENT APPLICATION**
published in accordance with Art. 153(4) EPC

(43) Date of publication:
**22.03.2017 Bulletin 2017/12**

(21) Application number: **15792651.0**

(22) Date of filing: **03.02.2015**

(51) Int Cl.:
*A61F 13/49* (2006.01)          *A61F 13/15* (2006.01)
*A61F 13/53* (2006.01)          *D04H 1/4374* (2012.01)
*D04H 1/492* (2012.01)

(86) International application number:
**PCT/JP2015/052957**

(87) International publication number:
**WO 2015/174106 (19.11.2015 Gazette 2015/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.05.2014 JP 2014098637**

(71) Applicant: **Unicharm Corporation**
**Ehime 799-0111 (JP)**

(72) Inventors:
• **KIMURA, Akihiro**
**Kanonji-shi**
**Kagawa 769-1602 (JP)**

• **DETANI, Ko**
**Kanonji-shi**
**Kagawa 769-1602 (JP)**
• **TANGE, Satoru**
**Kanonji-shi**
**Kagawa 769-1602 (JP)**
• **MATUMURA, Toshiaki**
**Kanonji-shi**
**Kagawa 769-1602 (JP)**
• **NOGUCHI, Atushi**
**Kanonji-shi**
**Kagawa 769-1602 (JP)**

(74) Representative: **Peter, Julian**
**Staeger & Sperling**
**Partnerschaftsgesellschaft mbB**
**Sonnenstrasse 19**
**80331 München (DE)**

(54) **ABSORBENT ARTICLE CONTAINING NON-WOVEN FABRIC SHEET FOR ABSORBENT, AND METHOD FOR PRODUCING NON-WOVEN FABRIC SHEET FOR USE IN SAID ABSORBENT ARTICLE**

(57)     The present invention provides: an absorbent article containing a non-woven fabric sheet for an absorbent, wherein the non-woven fabric sheet is produced without employing a strength-imparting means such as post-coating of an adhesive agent that can deteriorate a liquid diffusion property of the non-woven fabric sheet and therefore the non-woven fabric sheet has an excellent liquid diffusion property; and a method for producing the non-woven fabric sheet that can be used in the absorbent article. The absorbent article (1) according to the present invention comprises a liquid-permeable surface sheet (2), a liquid-impermeable back sheet (3), an absorbent (4) arranged between the surface sheet (2) and the back sheet (3), and a non-woven fabric sheet (8) for the absorbent (4), wherein the non-woven fabric sheet (8) contains pulp and comprises a pulp fiber layer (81) having a first surface and a second surface, a first-surface-side fiber layer (82) which is arranged on the first surface side of the pulp fiber layer (81) and is mainly composed of hydrophilic fibers having an average fiber length of 25 to 64 mm, and a second-surface-side fiber layer (83) which is arranged on the second surface side of the pulp fiber layer (81) and is mainly composed of hydrophilic fibers having an average fiber length of 25 to 64 mm.

**EP 3 143 976 A1**

**(Cont. next page)**

# FIG. 3

# FIG. 4

**Description**

Technical Field

The present invention relates to an absorbent article including a nonwoven fabric sheet for an absorbent body, and to a method for producing a nonwoven fabric sheet to be used in the absorbent article.

Background Art

Absorbent articles, such as disposable diapers, sanitary napkins and panty liners, are generally composed of a liquid-permeable front sheet, a liquid-impermeable back sheet and an absorbent body situated between the front sheet and the back sheet, and in particular, a core wrap covering super-absorbent polymer particles inside the absorbent body must have functions of reliably hold the super-absorbent polymer particles both when dry and when wet, and of rapidly diffusing body fluids such as urine or blood that have reached the absorbent article, over a wide area of the absorbent body, and rapidly absorbing the body fluids into the super-absorbent polymer particles.

For example, PTL 1 discloses an essentially longitudinal absorbent article having a liquid-permeable surface , a liquid-retaining absorbent body and a liquid-impermeable leak-resistant, the absorbent body having a diffusible absorption sheet and an absorption retaining sheet situated more to the back side than the diffusible absorption sheet, wherein (a) the diffusible absorption sheet comprises a sheet having a hydrophilicity (cosθ) of 0.5 to 1 and a Klemm water absorption rate of 40 [mm/min] or greater in the lengthwise direction, and (b) the absorption retaining sheet comprises a sheet made of fiber aggregates with a capillary osmotic pressure of 4000 to 15,000 [dyn/cm$^2$], which is a laminated sheet obtained by using a super-absorbent polymer having an absorption of 40 to 70 [g/g] for physiological saline and an absorption rate of 2 [ml/(0.3 g polymer·min)] or greater for the same, to sandwich the fiber aggregates at 10 to 100 wt% (see claim 1, etc.).

Citation List

Patent Literature

[PTL 1] Japanese Unexamined Patent Publication No. 4-89053

Summary of the Invention

Technical Problem

In the absorbent article disclosed in PTL 1, in order to maintain its strength, the diffusible absorption sheet of the absorbent body is formed by either bonding the fibers using polyvinyl alcohol or the like as the binder, or by fusing the fibers together by a spunbond method, and therefore the fluid diffusibility is sometimes impeded by the bonded sections or fused sections.

It is therefore an object of the present invention to provide an absorbent article including a nonwoven fabric sheet for an absorbent body having excellent fluid diffusibility, without using strengthening means by post-coating of an adhesive which can impede fluid diffusibility, as well as a method for producing a nonwoven fabric sheet for an absorbent body to be used in the absorbent article.

Solution to Problem

The absorbent article of the present invention is an absorbent article including a liquid-permeable front sheet, a liquid-impermeable back sheet, an absorbent body situated between the front sheet and the back sheet, and a nonwoven fabric sheet for the absorbent body, wherein the nonwoven fabric sheet includes a pulp fiber layer that includes pulp and has a first surface and a second surface, a first surface side fiber layer situated on the first surface side of the pulp fiber layer and including mainly hydrophilic fibers with a mean fiber length of 25 mm to 64 mm, and a second surface side fiber layer situated on the second surface side of the pulp fiber layer and including mainly hydrophilic fibers with a mean fiber length of 25 mm to 64 mm.

According to the absorbent article of the present invention, the nonwoven fabric sheet for the absorbent body comprising 3 or more layers including a pulp fiber layer, a first surface side fiber layer and a second surface side fiber layer is formed by tangling the fibers together by a high-pressure stream jet without using strengthening means by post-coating of an adhesive or the like, which can impede fluid diffusibility, and therefore fluids such as body fluids can be widely and rapidly diffused in an in-plane direction of the nonwoven fabric sheet by utilizing capillary action of the fiber aggregates forming the first surface side fiber layer and the second surface side fiber layer. In addition, since the first surface side fiber layer and second surface side fiber layer are situated on both surface sides of the pulp fiber layer, fluid that has diffused in the in-plane direction of the first surface side fiber layer and second surface side fiber layer can be absorbed from both sides of the pulp fiber layer, allowing the water capacity of the nonwoven fabric sheet to be drastically increased while also allowing long-lasting fluid diffusion to be achieved in the in-plane direction of the nonwoven fabric sheet.

Advantageous Effects of Invention

According to the present invention it is possible to provide an absorbent article including a nonwoven fabric sheet for an absorbent body having excellent fluid diffusibility, and a method for producing a nonwoven fabric sheet for an absorbent body to be used in the absorbent article.

Brief Description of Drawings

Fig. 1 is a perspective view (schematic view) of a disposable diaper as an embodiment of the absorbent article of the present invention.
Fig. 2 is a plan view of the disposable diaper of Fig. 1 in the deployed state.
Fig. 3 is a cross-sectional view in the widthwise direction along line III-III' of Fig. 2.
Fig. 4 is a cross-sectional view (schematic view) of a nonwoven fabric sheet according to an embodiment of the present invention.

Description of Embodiments

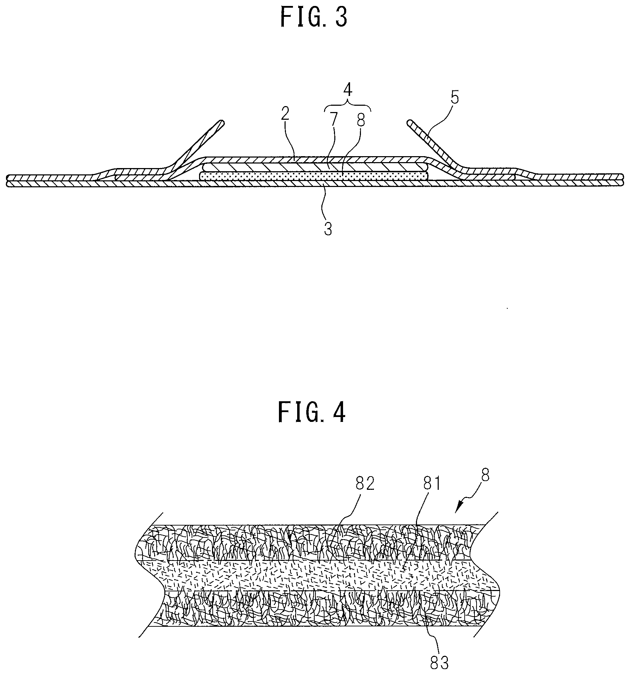

Preferred embodiments of the absorbent article of the present invention will now be described in detail with reference to the accompanying drawings.

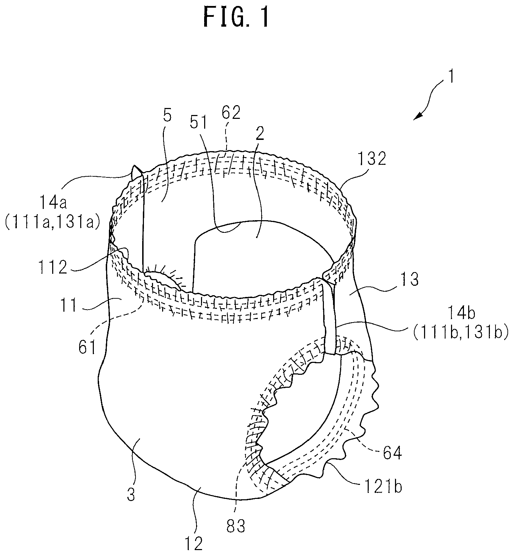

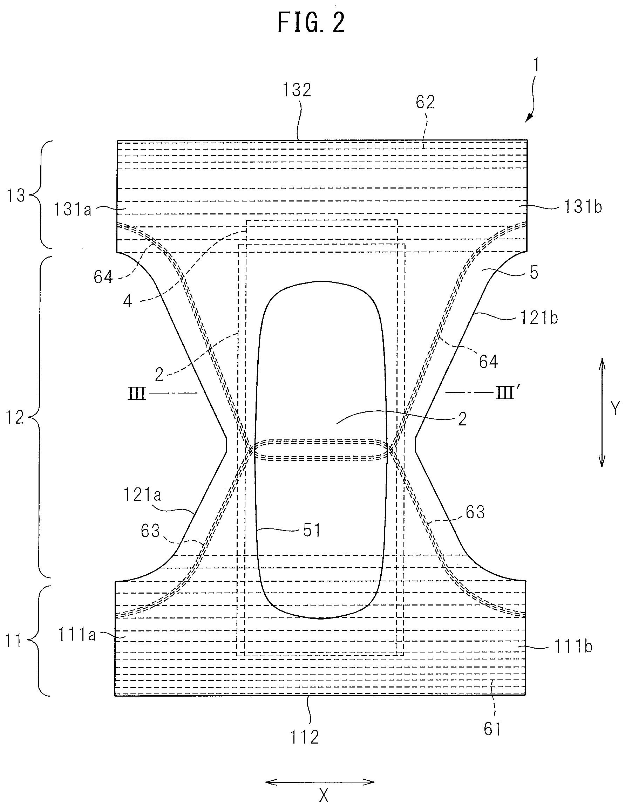

Fig. 1 is a perspective view (schematic view) of a disposable diaper as an embodiment of the absorbent article of the present invention, and Fig. 2 is a plan view of the disposable diaper of Fig. 1 in the deployed state. As shown in Fig. 1 and Fig. 2, the disposable diaper 1 as an embodiment of the present invention has a front section 11 which is contacted with the abdominal region of the wearer, a middle section 12 which is contacted with the crotch region of the wearer, and a back section 13 which is contacted with the gluteal region and/or back region of the wearer. As shown in Fig. 1, both edges 111 a, 111 b of the front section 11 and both edges 131 a, 131 b of the back section 13 are joined together at the joining sections 14a, 14b, whereby a waist opening is formed by the edge 112 of the front section 11 and the edge 132 of the back section 13 and leg openings are formed by both edges 121a, 121 b of the middle section 12, the disposable diaper 1 having a pants-type shape.
As shown in Fig. 1 and Fig. 2, the disposable diaper 1 comprises a front sheet 2 made of a liquid-permeable nonwoven fabric sheet, plastic film or the like, a back sheet 3 made of a liquid-impermeable polyethylene film or the like, an absorbent body 4 provided between the front sheet 2 and the back sheet 3, a liquid-impermeable cover sheet 5, and elastic members 61, 62, 63, 64. The cover sheet 5 provided on the skin side surface of the front sheet 2 has an opening 51 formed at approximately the center, a portion of the front sheet 2 (the portion of the region where the absorbent body 4 is situated) being exposed from the opening 51 of the cover sheet 5 and, together with the cover sheet 5, forming the skin side surface of the disposable diaper 1.
Also, as shown in Fig. 1 and Fig. 2, elastic members 61, 62, 63, 64 are provided between the back sheet 3 and the cover sheet 5, which have hourglass shapes of approximately equal dimensions. Elastic contractive force of the elastic members 61, 62 causes formation of waist gathers at the waist opening, and elastic contractive force of the elastic members 63, 64 causes formation of leg gathers (leg side cuffs) at the leg openings. The leg gathers can prevent leakage of excreta from the leg openings. Throughout the present description, the widthwise direction X is the widthwise direction of the disposable diaper 1 (absorbent article) in the deployed state in a plane view (the short direction), and the lengthwise direction Y is the lengthwise direction of the disposable diaper 1 (absorbent article) in the deployed state in a plane view (the front-back direction of the wearer), the widthwise direction X and lengthwise direction Y being mutually orthogonal in a plane view.
An absorbent body to be used in a disposable diaper (absorbent article) according to an embodiment of the present invention, and a nonwoven fabric sheet for the absorbent body, will now be described in detail. Fig. 3 is a cross-sectional view in the widthwise direction along line III-III' of Fig. 2. As shown in Fig. 3, the absorbent body 4 of this embodiment includes an absorbent core 7 made of a water-absorbing material, a nonwoven fabric sheet 8 for an absorbent body situated on the back sheet 3 side of the absorbent core 7, and a core wrap (not shown) that encloses the absorbent core 7 and the nonwoven fabric sheet 8. According to the present invention, the nonwoven fabric sheet may be situated in the sheet of the core wrap enclosing the absorbent core, or it may be situated in contact with the core wrap on the outer side of the core wrap; however, situating the nonwoven fabric sheet in the sheet of the core wrap enclosing the absorbent core is advantageous for absorption and diffusion of fluids such as body fluids, and therefore the nonwoven fabric sheet is preferably situated in the sheet of the core wrap enclosing the absorbent core. Throughout the present description, "for the absorbent body" means, in an absorbent body including an absorbent core and a core wrap enclosing the absorbent core, for placement in contact with the absorbent core, or for placement in contact with the core wrap on the outer side of the core wrap. Also according to the present invention, in order to further improve the diffusibility of fluids such as body fluids passing through the front sheet, the nonwoven fabric sheet or a fluid diffusing sheet different from the nonwoven fabric sheet may also be situated on the front sheet side of the absorbent core.

The absorbent core is not particularly restricted, and for example, one may be used that has a super-absorbent polymer dispersed and held in fiber aggregates of fluff pulp or a nonwoven fabric. The super-absorbent polymer has a three-dimensional network structure with appropriate crosslinking of a water-soluble polymer, and it can absorb water at up to 20 or more times its own weight. Examples of such super-absorbent polymers include starch-based polymers, crosslinked carboxymethylated cellulose, acrylic acid-based polymers including polymers or copolymers of acrylic acid or alkali metal salt acrylates, and amino acid-based polymers.

According to the present invention, the nonwoven fabric sheet for an absorbent body is constructed with 3 or more layers including a pulp fiber layer that includes pulp and has a first surface and a second surface, a first surface side fiber layer situated on the first surface side of the pulp fiber layer and including mainly hydrophilic fibers with a mean fiber length of 25 mm to 64 mm, and a second surface side fiber layer situated on the second surface side of the pulp fiber layer and including mainly hydrophilic fibers with a mean fiber length of 25 mm to 64 mm.

According to the present invention, the phrase "including mainly hydrophilic fibers" means including hydrophilic fibers at greater than 50 mass% with respect to the total mass. The phrase may therefore be reworded to mean including hydrophilic fibers at greater than 50 mass% and up to 100 mass%, while including other components (hydrophobic fibers, for example) at from 0 mass% to less than 50 mass%.

Fig. 4 is a cross-sectional view (schematic view) of a nonwoven fabric sheet according to an embodiment of the present invention. The nonwoven fabric sheet 8 of this embodiment is formed of a layered body having a three-layer structure, including a pulp fiber layer 81 with a first surface and a second surface, a first surface side fiber layer 82 situated on the first surface side of the pulp fiber layer 81, and a second surface side fiber layer 83 situated on the second surface side of the pulp fiber layer.

The pulp fiber layer of the present invention includes mainly pulp which is generally cellulosic fibers with fiber lengths of 1 to 10 mm (i.e., it includes it in a proportion of greater than 50 mass%), and is preferably formed of 100 mass% pulp. The type of pulp is not particularly restricted, and any desired pulp such as wood pulp, nonwood pulp or recycled pulp may be used; however, fluff pulp is most preferably used from the viewpoint of high ability to draw out fluids such as body fluids that are diffused in the first surface side fiber layer and second surface side fiber layer, from the first surface side fiber layer and second surface side fiber layer.

The pulp preferably includes pulp with a Kajaani mean fiber length of 2 to 3 mm, from the viewpoint of water absorbing property, water retention, flexibility and handleability. The "Kajaani mean fiber length" is the length-weighted average fiber length measured with a Kajaani fiber length analyzer by Kajaani Automation Co., according to JAPAN TAPPI wood pulp test method No.52:2000. Also, the basis weight of the pulp is not particularly restricted but is preferably about 5 to 60 g/m$^2$ and more preferably about 15 to 40 g/m$^2$, from the viewpoint of water retention, flexibility and bulk.

The first surface side fiber layer and second surface side fiber layer of the present invention includes mainly hydrophilic fibers with a mean fiber length of 25 mm to 64 mm (i.e., it includes them at greater than 50 mass%), and it preferably includes them at 70 mass% or greater. There are no particular restrictions on the hydrophilic fibers, but from the viewpoint of fluid diffusibility, strength, flexibility and general utility, examples include cellulosic fibers, and more specifically, natural fibers such as cotton and regenerated fibers such as rayon and cupra. Synthetic fibers (such as rayon fibers) that have been hydrophilically treated on the surfaces may also be used. Of these, rayon fibers are especially preferred for use from the viewpoint of fluid diffusibility, strength after tangling, handleability, and general utility.

According to the present invention, the hydrophilic fibers preferably have a mean fiber length in the range of 25 mm to 64 mm. If the mean fiber length is within this range, fluids such as body fluids that have been discharged can be widely and rapidly diffused in the in-plane direction of the nonwoven fabric sheet utilizing capillary action of the fiber aggregates forming the first surface side fiber layer and the second surface side fiber layer, and diffused fluids can be easily delivered to the pulp fiber layer situated between the first surface side fiber layer and the second surface side fiber layer. The "mean fiber length" is the mean fiber length measured according to "A7.1.1, Method A (Standard method) Method for measuring lengths of individual fibers on graduated glass plate", under "A7.1 Fiber length measurement" in appendix A of JIS L 1015:2010. This method is the test method corresponding to ISO 6989 published in 1981.

The forms of the hydrophilic fibers are not particularly restricted, and they may have ordinary circular cross-sections, or irregular cross-sections that are Y-shaped, cross-shaped, hollow or the like, or combinations of these forms.

When the hydrophilic fibers include fibers with irregular cross-sections, the fibers with irregular cross-sections will have large surface areas and excellent liquid absorption, thereby allowing the fluid diffusibility of the fiber aggregates forming the first surface side fiber layer and second surface side fiber layer to be further increased.

According to the present invention, the first surface side fiber layer and second surface side fiber layer may include components other than the hydrophilic fibers in proportions lower than that of the hydrophilic fiber in terms of mass ratio (i.e., proportions of less than 50 mass%), and they are preferably proportions of no greater than 30 mass%. Such other components include hydrophobic fibers, heat-sealable fibers, various treatment agents and fillers, used appropriately either alone or in combinations depending on the desired fluid diffusibility, strength, flexibility, water retention, production cost, etc.

As hydrophobic fibers there may be used, for example, thermoplastic fibers such as polyethylene, polypropylene, nylon and polyester, or composite fibers with combinations of these thermoplastic fibers; however, polyester-based fibers such as polyethylene terephthalate are preferably used from the viewpoint of strength when wet, and bulk, flexibility and the like.

Also, from the viewpoint of fluid diffusibility, the hydrophobic fibers used are preferably fibers subjected to hydrophilic treatment on the surfaces with a hydrophilic lubricant or the like, and more preferably polyester-based fibers subjected to hydrophilic treatment. The hydrophilic lubricant to be used for the hydrophilic treatment is not particularly restricted, and examples include alkyl phosphate ester salts and alkyl phosphate metal salts. Moreover, according to the present invention, the first surface side fiber layer, pulp fiber layer and second surface side fiber layer are integrated by a high-pressure stream jet such as a water jet as described below, and therefore the surfaces of the hydrophobic fibers are most preferably subjected to hydrophilic treatment using a hydrophilic lubricant having durability of a level such that it is not flushed off by the high-pressure stream jet (a resistant hydrophilic lubricant). Such a resistant hydrophilic lubricant is not particularly restricted, and for example, it may be a polyether ester, ether nonion, polyether-modified silicone, sulfosuccinate, polyoxyethyleneamide ether, alkylimidazoline-type cation, polyglycerin polyester, a C10-30 betaine compound or sulfuric acid ester salt or sulfonate salt mixed with a C10-30 alkyl phosphate ester salt, of a mixed lubricant such as a mixture of an alkyl phosphate ester salt and a polyether-modified silicone.

Also, the heat-sealable fibers may be ones including a low-melting-point thermoplastic resin such as a polyethylene resin or low melting point polypropylene at least on their surfaces, examples including polyethylene resin single component fibers; polypropylene resin single component fibers; core-sheath composite synthetic fibers with a polyethylene terephthalate resin core and a polyethylene resin sheath; core-sheath composite synthetic fibers with a polypropylene resin core and a polyethylene resin sheath; core-sheath composite synthetic fibers with a high melting point polypropylene resin core and a low melting point polypropylene resin sheath; side-by-side composite synthetic fibers composed of a polyethylene terephthalate resin and a polyethylene resin; and side-by-side composite synthetic fibers composed of a polypropylene resin and a polyethylene resin.

When the first surface side fiber layer and the second surface side fiber layer include heat-sealable fibers, the first surface side fiber layer, pulp fiber layer and second surface side fiber layer are integrated by a high-pressure stream jet such as a water jet as described below to obtain a nonwoven fabric sheet, after which the nonwoven fabric sheet is heat set to melt the low melting point resin of the heat-sealable fibers and fuse them with the other fibers, thereby allowing the strength, and especially the wet strength, of the nonwoven fabric sheet to be increased. Since the heat-sealable fibers are included in a lower proportion than the hydrophilic fibers based on mass ratio, even when fused sections are formed by the heat-sealable fibers, the fluid diffusibility of the nonwoven fabric sheet is not impeded and the strength of the nonwoven fabric sheet can be reinforced in an auxiliary manner.

The first surface side fiber layer and second surface side fiber layer may employ a fiber web formed by a method such as an airlaid method; however, the fiber layer of at least the first surface side fiber layer or the second surface side fiber layer is preferably a carded web formed using a carding machine. By using a carded web it is possible to adequately tangle together the fibers of each fiber layer and the fibers within each fiber layer, when the first surface side fiber layer, pulp fiber layer and second surface side fiber layer are integrated by a high-pressure stream jet such as a water jet as described below, even when the fiber lengths of the constituent fibers are long. The form of the carded web is not particularly restricted and may be any form such as a parallel web, cross web, random web or the like.

The first surface side fiber layer and second surface side fiber layer of the present invention may be fiber layers having the same construction (i.e., fiber layers having the same fiber type, content and layer structure), or fiber layers with different constructions (i.e., fiber layers differing in at least one aspect from among fiber type, content and layer structure).

A method for producing a nonwoven fabric sheet for an absorbent body to be used in an absorbent article of the present invention will now be described. The nonwoven fabric sheet can be obtained by a production method that includes at least the following steps; a step of supplying a fiber web for the second surface side fiber layer including hydrophilic fibers, a step of supplying pulp for the pulp fiber layer including hydrophilic fibers onto the fiber web, a step of supplying a fiber web for the first surface side fiber layer including hydrophilic fibers onto the pulp, to obtain a layered body, and a step of high-pressure stream jet treatment from both surface sides of the layered body to tangle together the fibers of each of the fiber layers. More specifically, the constituent fibers such as hydrophilic fibers may be treated with a carding machine or the like either directly or after being blended in a prescribed mixing proportion, to prepare a fiber web for the second surface side fiber layer, such as a carded web, and then the prepared fiber web conveyed while supplying pulp for the pulp fiber layer, including hydrophilic fibers such as fluff pulp, onto the fiber web by airlaying or the like, and further supplying a fiber web for the first surface side fiber layer including hydrophilic fibers such as carded web, onto the pulp to obtain a layered body, and then tangling together at least the fibers of each fiber layer by high-pressure stream jet treatment such as a water jet from both surface sides of the layered body, to obtain a nonwoven fabric sheet in which the first surface side fiber layer, the pulp fiber layer and the second surface side fiber layer are integrated. Also, when the first surface side fiber layer and the second surface side fiber layer include heat-sealable fibers, the nonwoven fabric sheet obtained in this manner may be further heat set to obtain a nonwoven fabric sheet with reinforced sheet strength.

The nonwoven fabric sheet obtained in this manner has a structure in which the fibers in each fiber layer and the fibers between each fiber layer are tangled by a high-pressure stream jet such as a water jet, and therefore it is possible to impart excellent sheet strength and fluid diffusibility, while also promoting delivery to the pulp fiber layer of the fluids diffused in the in-plane direction of the nonwoven fabric sheets of the first surface side fiber layer and second surface side fiber layer. Furthermore, since the first surface side fiber layer and the second surface side fiber layer can reabsorb fluids and diffuse them in the in-plane direction after having delivered the fluids to the pulp fiber layer, the cycle of absorption of fluids, diffusion in the in-plane direction and delivery to the pulp fiber layer can be carried out repeatedly in the nonwoven fabric sheet, and long-lasting diffusion of the fluids allows the fluid diffusion region and the amount of fluids held in the nonwoven fabric sheet to be markedly increased.

Moreover, if the structure is such that some of the constituent fibers in the fiber layer of either or both the first surface side fiber layer and second surface side fiber layer are incorporated in the interior of the pulp fiber layer by the high-pressure stream jet such as a water jet, then fluids that have diffused in the in-plane direction of the nonwoven fabric sheets of the first surface side fiber layer and second surface side fiber layer can be more easily delivered to the pulp fiber layer, and thus the cycle of absorption of fluids, diffusion in the in-plane direction and delivery to the pulp fiber layer in the nonwoven fabric sheet can be accomplished more rapidly, as a result allowing the diffusion rate and diffusion region (diffusion area) of fluids in the nonwoven fabric sheet to be further increased.

According to the present invention, the nonwoven fabric sheet for an absorbent body has a fluid absorption height of preferably 110 mm or greater, more preferably 120 mm or greater and even more preferably 130 mm or greater, in a water absorption test based on the Klemm method described below. Moreover, the nonwoven fabric sheet has a water absorption factor of preferably 2.0 times or greater, more preferably 2.4 times or greater and even more preferably 3.0 times or greater, in the water absorption test. If the fluid absorption height and water absorption factor are within these ranges, fluids such as body fluids can be widely and rapidly diffused in the in-plane direction of the nonwoven fabric sheet, while fluids can also rapidly close up the pulp fiber layer interior to match the height of the liquid permeability.

Furthermore, according to the present invention, the nonwoven fabric sheet preferably has a fluid absorption height in the fiber layer of either or both the first surface side fiber layer and second surface side fiber layer, that is higher than the fluid absorption height of the pulp fiber layer. If the fluid absorption heights of each of the fiber layers have this relationship, then in the aforementioned cycle, the first surface side fiber layer or the second surface side fiber layer will more easily deliver fluids to the regions of the pulp fiber layer where fluids have not yet been absorbed or retained, and therefore diffusion of fluids can be accomplished in a more long-lasting and rapid manner, and the fluid diffusion regions and water retention capacity of the nonwoven fabric sheet can be further increased.

The water absorption test based on the Klemm method allows measurement based on the water absorption test method of JIS P 8141:2004, and the specific procedure is explained in the examples provided below.

According to the present invention, from the viewpoint of fluid diffusibility, sheet strength, liquid permeability, etc., the basis weight of the nonwoven fabric sheet is preferably a basis weight of 15 to 100 g/m$^2$, more preferably a basis weight of 20 to 80 g/m$^2$, and even more preferably a basis weight of 30 to 60 g/m$^2$. Also, from the viewpoint of the state of tangling between the fibers, the fluid diffusibility, the sheet strength, the liquid permeability, etc., the basis weight of each fiber layer composing the nonwoven fabric sheet is preferably in the range of about 10 to 60 g/m$^2$ for the pulp fiber layer, and preferably in the range of about 5 to 50 g/m$^2$ for each of the first surface side fiber layer and the second surface side fiber layer.

Also, the density of the nonwoven fabric sheet is preferably 50 to 300 mg/cm$^3$, more preferably 50 to 200 mg/cm$^3$ and even more preferably 60 to 150 mg/cm$^3$, in consideration of the state of capillary formation necessary for diffusion of fluids, the sheet strength, etc.

According to the present invention, the nonwoven fabric sheet may be used as a member that can contact with the absorbent body of the absorbent article, and for example, it may be used in the absorbent body as a diffusing sheet to be situated on the front sheet side and/or back sheet side of the absorbent core, or as a core wrap to cover the absorbent core. When the nonwoven fabric sheet is used as such a member, fluids such as body fluids that have been discharged are diffused in a wide range throughout the nonwoven fabric sheet, and it can therefore absorb fluids in a wide region of the absorbent core that is adjacent to the nonwoven fabric sheet, and furthermore when the nonwoven fabric sheet is situated on the back sheet side of the absorbent core, it can notably reduce the rewetting amount in the absorbent article (for example, a reduction of 20% or more).

The present invention can be applied not only to a disposable diaper as with this embodiment, but also to various types of absorbent articles, such as incontinence pads, sanitary napkins and panty liners. Moreover, the absorbent article of the present invention is not restricted to the embodiment described above or the examples described below, and can be appropriately modified within a range that is not outside of the object and gist of the present invention.

Examples

The present invention will now be explained in more detail based on examples, with the understanding that the present invention is not limited to the examples.

Example 1

There were provided rayon fibers (product of Daiwabo Rayon Co., Ltd.) as hydrophilic fibers and polyethylene terephthalate (PET) fibers (product of Toyobo, Ltd.) having the surfaces hydrophilically treated with a resistant hydrophilic lubricant, as hydrophobic fibers, and the rayon fibers and PET fibers were blended to rayon fiber/PET fibers = 70 mass%/30 mass%, after which a carding machine was used to fabricate a carded web with a basis weight of 12.0 $g/m^2$, for use as the second surface side fiber layer. The fabricated carded web was conveyed while continuously supplying fluff pulp (NB416 by Weyerhaeuser Co., Ltd) for the pulp fiber layer onto the carded web to a basis weight of 21.0 $g/m^2$, forming a pulp fiber layer on the second surface side fiber layer. Next, a carded web fabricated in the same manner as the second surface side fiber layer was supplied onto the pulp fiber layer to form the first surface side fiber layer, thus obtaining a layered body comprising the first surface side fiber layer, pulp fiber layer and second surface side fiber layer. The layered body obtained in this manner was conveyed at a transport speed of 10 m/min, while both surface sides of the layered body were subjected to high-pressure stream jet treatment with a water jet (first surface side fiber layer side water pressure: 2 to 5 MPa, second surface side fiber layer side water pressure: 3 MPa, nozzle orifice diameter: 92 $\mu$m, nozzle pitch: 0.5 mm, 2 rows) to tangle the constituent fibers in each fiber layer and between each fiber layer, thereby obtaining a three-layer structure nonwoven fabric sheet in which the first surface side fiber layer, pulp fiber layer and second surface side fiber layer were integrated.

Example 2

A three-layer structure nonwoven fabric sheet was obtained by production in the same manner as Example 1, except that the fluff pulp for the pulp fiber layer was supplied onto the carded web to a basis weight of 26.0 $g/m^2$.

Example 3

A three-layer structure nonwoven fabric sheet was obtained by production in the same manner as Example 2, except that each carded web forming the first surface side fiber layer and the second surface side fiber layer was fabricated without using hydrophobic fibers (PET fibers).

Comparative Example 1

There were provided rayon fibers (product of Daiwabo Rayon Co., Ltd.) as hydrophilic fibers and polyethylene terephthalate (PET) fibers (product of Toyobo, Ltd.) having the surfaces hydrophilically treated with a hydrophilic lubricant, as hydrophobic fibers, and the rayon fibers and PET fibers were blended to rayon fiber/PET fibers = 30 mass%/70 mass%, after which a carding machine was used to fabricate a carded web with a basis weight of 22.5 $g/m^2$, for use as a fiber layer corresponding to the second surface side fiber layer of the present invention. The fabricated carded web was conveyed while supplying a carded web fabricated in the same manner as the fiber layer corresponding to the second surface side fiber layer, onto the carded web, forming a fiber layer corresponding to the first surface side fiber layer of the present invention, to obtain a layered body comprising two fiber layers. The layered body obtained in this manner was conveyed at a transport speed of 10 m/min, while both surface sides of the layered body were subjected to high-pressure stream jet treatment with a water jet in the same manner as Example 1 to tangle the constituent fibers in each fiber layer and between each fiber layer, thereby obtaining a two-layer structure nonwoven fabric sheet in which the fiber layer corresponding to the first surface side fiber layer and the fiber layer corresponding to the second surface side fiber layer were integrated.

Comparative Example 2

A two-layer structure nonwoven fabric sheet was obtained in the same manner as Comparative Example 1, except that the carded web forming each fiber layer was fabricated by blending with rayon fiber/PET fibers = 50 mass%/50 mass%.

Comparative Example 3

A two-layer structure nonwoven fabric sheet was obtained in the same manner as Comparative Example 1, except that the carded web forming each fiber layer was fabricated by blending with rayon fiber/PET fibers = 70 mass%/30 mass%.

For each of the nonwoven fabric sheets of Examples 1 to 3 and Comparative Examples 1 to 3 obtained as described above, the basis weight (g/m$^2$), thickness (mm), density (g/cm$^3$), fluid absorption height (mm) after 5 minutes in the Klemm method and transported amount per unit mass (g/g) were measured by the following measuring methods. Also, the mixing ratio of rayon fibers and PET fibers in the first surface side fiber layer and second surface side fiber layer was measured by the measuring method described below, using each carded web fabricated in the nonwoven fabric sheet production process described above. The measurement results are shown in Table 1.

[Table 1]

Table 1

| | | Example 1 | Example 2 | Example 3 | Comparative Example 1 | Comparative Example 2 | Comparative Example 3 |
|---|---|---|---|---|---|---|---|
| Starting material composition of nonwoven fabric sheet | First surface side fiber layer | Rayon/PET 70%/30% (Set basis weight 12.0 g/m$^2$) | Rayon/PET 70%/30% (Set basis weight 12.0 g/m$^2$) | Rayon 100% (Set basis weight 12.0 g/m$^2$) | Rayon/PET 30%/70% (Set basis weight 22.5 g/m$^2$) | Rayon/PET 50%/50% (Set basis weight 22.5 g/m$^2$) | Rayon/PET 70%/30% (Set basis weight 22.5 g/m$^2$) |
| | Pulp fiber layer | Pulp 100% (Set basis weight 21.0 g/m$^2$) | Pulp 100% (Set basis weight 26.0 g/m$^2$) | Pulp 100% (Set basis weight 26.0 g/m$^2$) | - | - | - |
| | Second surface side fiber layer | Rayon/PET 70%/30% (Set basis weight 12.0 g/m$^2$) | Rayon/PET 70%/30% (Set basis weight 12.0 g/m$^2$) | Rayon 100% (Set basis weight 12.0 g/m$^2$) | Rayon/PET 30%/70% (Set basis weight 22.5 g/m$^2$) | Rayon/PET 50%/50% (Set basis weight 22.5 g/m$^2$) | Rayon/PET 70%/30% (Set basis weight 22.5 g/m$^2$) |
| Basis weight (g/m$^2$) | | 44.2 | 51.3 | 50.9 | 45.8 | 46.1 | 45.1 |
| Thickness (mm) | | 0.57 | 0.57 | 0.48 | 0.62 | 0.57 | 0.53 |
| Density (mg/cm$^3$) | | 78 | 90 | 106 | 74 | 81 | 85 |
| Fluid absorption height (mm) | | 123 | 135 | 157 | 8 | 37 | 69 |
| Transported amount (g/g) | | 3.38 | 4.11 | 4.59 | 0.22 | 1.19 | 1.91 |

As shown in Table 1, the nonwoven fabric sheets of Examples 1 to 3 all had fluid absorption heights of 120 mm or greater and transport amounts of 3 g/g or greater, exhibiting higher fluid absorption heights and transported amounts than the nonwoven fabric sheets of Comparative Examples 1 to 3, and having excellent fluid diffusibility. Also, based on comparison of Examples 1 to 3, it was demonstrated that a higher rayon fiber mixing ratio in the first surface side fiber layer and second surface side fiber layer results in a higher fluid absorption height and transported amount being exhibited, and increased fluid diffusibility of the nonwoven fabric sheet corresponding to the rayon fiber mixing ratio.

The methods for measuring the properties were as follows.

[Basis weight]

Ten samples of size of 100 mm × 100 mm are taken and the mass of each sample is measured. Next, the mass (g) of each sample is divided by the area ($m^2$) of each sample to calculate the basis weight ($g/m^2$) of each sample. The mean value for the basis weights of the 10 samples is calculated, and the mean value is used as the basis weight.

[Thickness]

The thickness of the nonwoven fabric is measured using a THICKNESS GAUGE UF-60 by Daiei Kagaku Seiki Mfg. Co., Ltd. Measurement of the thickness with the UF-60 is carried out with a 44 mm diameter measuring surface, and application of 0.3 kPa pressure on the nonwoven fabric.

[Density]

The density of the nonwoven fabric is calculated by dividing the basis weight of the nonwoven fabric by the thickness.

[Fluid absorption height and transported amount]

The fluid absorption height and transported amount after 5 minutes in the Klemm method are measured according to the water absorption test method of JIS P 8141:2004, in the following manner.

(1) The sample is cut to a size of 230 mm × 25 mm (length × width), a line is drawn 30 mm from the edge in the lengthwise direction, and the initial mass ($W_0$) of the test strip is measured.
(2) A cuboid dipping container with dimensions 170 mm × 90 mm × 40 mm (length × width × depth) is filled with artificial urine to a height of 35 mm. The artificial urine is prepared by dissolving 200 g of urea, 80 g of sodium chloride, 8 g of magnesium sulfate, 3 g of calcium chloride and approximately 1 g of dye: Blue #1 in 10 L of ion-exchanged water.
(3) The test strip is fixed to a suspending member with the drawn line at the lower end and dipped in the artificial urine up to the drawn line, and allowed to stand for 5 minutes.
(4) After standing for 5 minutes, the height increase of the artificial urine from the drawn line is measured as the fluid absorption height (mm).
(5) Next, the test strip is removed from the suspending member, the section with a length of 30 mm (the section below the drawn line) that was dipped in the artificial urine is cut out, and the mass ($W_1$) of the remaining section with a length of 200 mm is measured.
(6) The transported amount (X) (g/g) is calculated by the following formula.

$$X = \{(W_1 \times 230/200)-W_0\}/W_0$$

(7) The above test is repeated 5 times, and the mean value is used.

[Mixing ratio of rayon fibers and PET fibers]

The mixing ratio of rayon fibers and PET fibers in the carded web is determined by measuring the mixing ratio of rayon fibers by the following procedure.

(1) A fiber web including rayon fibers and PET fibers is prepared as a measuring sample.
(2) An approximately 0.2 g portion of the fiber web is cut to a size fitting in a 100 mL beaker, and the mass (g) of the cut fiber web (sample initial mass $W_A$) is measured.

(3) The cut fiber web and 30 g of hexafluoroisopropanol are placed in a 100 mL beaker, and stirred for 1 hour at a rotational speed of 300 rpm.

(4) The contents of the beaker are filtered using filter paper with a premeasured mass (g) (filter paper initial mass $W_F$).

(5) The residue on the filter paper is allowed to stand in a draft chamber for each filter paper, and dried for 1 hour.

(6) The masses (g) of the residue and filter paper after drying are measured.

(7) The mass (g) of the rayon fibers in the fiber web (rayon fiber mass $W_R$) is calculated by subtracting the masses of the residue and filter paper after drying from the total of the sample initial mass $W_A$ and the filter paper initial mass $W_F$.

(8) The rayon fiber mixing ratio $F_A$ (mass%) is calculated from the calculated rayon fiber mass and sample initial mass $W_A$, using the following formula.

$$F_A = (W_R/W_A) \times 100$$

Exemplary aspects of the present invention will now be described.

One aspect of the present invention (aspect 1) is an absorbent article including a liquid-permeable front sheet, a liquid-impermeable back sheet, an absorbent body situated between the front sheet and the back sheet, and a nonwoven fabric sheet for an absorbent body, wherein the nonwoven fabric sheet includes a pulp fiber layer that includes pulp and has a first surface and a second surface, a first surface side fiber layer situated on the first surface side of the pulp fiber layer and including mainly hydrophilic fibers with a mean fiber length of 25 mm to 64 mm, and a second surface side fiber layer situated on the second surface side of the pulp fiber layer and including mainly hydrophilic fibers with a mean fiber length of 25 mm to 64 mm.

According to another aspect of the present invention (aspect 2), in the absorbent article of aspect 1, the fiber layer of either or both the first surface side fiber layer and second surface side fiber layer further includes hydrophobic fibers in a smaller amount than the hydrophilic fibers, as the mass ratio.

According to yet another aspect of the present invention (aspect 3), in the absorbent article of aspect 2, the hydrophobic fibers include thermoplastic fibers.

According to yet another aspect of the present invention (aspect 4), in the absorbent article of aspect 3, the thermoplastic fibers are hydrophilically treated polyester-based fibers.

According to yet another aspect of the present invention (aspect 5), in the absorbent article of any one of aspects 1 to 4, the hydrophilic fibers in the fiber layer of either or both the first surface side fiber layer and second surface side fiber layer have irregular cross-sections.

According to yet another aspect of the present invention (aspect 6), in the absorbent article of any one of aspects 1 to 5, the hydrophilic fibers in the fiber layer of either or both the first surface side fiber layer and second surface side fiber layer include cellulosic fibers.

According to yet another aspect of the present invention (aspect 7), in the absorbent article of any one of aspects 1 to 6, the pulp in the pulp fiber layer includes fluff pulp.

According to yet another aspect of the present invention (aspect 8), in the absorbent article of any one of aspects 1 to 7, the pulp in the pulp fiber layer includes pulp with a Kajaani mean fiber length of 2 to 3 mm.

According to yet another aspect of the present invention (aspect 9), in the absorbent article according to any one of aspects 1 to 8, there is included a structure in which the fibers of each of the fiber layers are tangled together by the action of a water stream.

According to yet another aspect of the present invention (aspect 10), in the absorbent article of any one of aspects 1 to 9, the fiber layer of either or both the first surface side fiber layer and second surface side fiber layer includes a carded web.

According to yet another aspect of the present invention (aspect 11), in the absorbent article of any one of aspects 1 to 10, there is included a structure in which a portion of the fibers in the fiber layer of either or both the first surface side fiber layer and second surface side fiber layer are incorporated inside the pulp fiber layer.

According to yet another aspect of the present invention (aspect 12), in the absorbent article according to any one of aspects 1 to 11, the fluid absorption height of the fiber layer of either or both the first surface side fiber layer and the second surface side fiber layer is higher than the fluid absorption height of the pulp fiber layer, in a water absorption test by the Klemm method.

According to yet another aspect of the present invention (aspect 13), in the absorbent article according to any one of aspects 1 to 12, the absorbent body includes an absorbent core, and the nonwoven fabric sheet is situated at least between the back sheet and the absorbent core.

Yet another aspect of the present invention (aspect 14) is a method for producing a nonwoven fabric sheet for an absorbent body to be used in an absorbent article according to any one of aspects 1 to 13, the method including a step of supplying a fiber web for the second surface side fiber layer including hydrophilic fibers, a step of supplying pulp for the pulp fiber layer including hydrophilic fibers onto the fiber web, a step of supplying a fiber web for the first surface side fiber layer including hydrophilic fibers onto the pulp, to obtain a layered body, and a step of high-pressure stream jet treatment from both surface sides of the layered body to tangle together at least the fibers of each of the fiber layers.

Reference Sign List

| | |
|---|---|
| 1 | Disposable diaper |
| 2 | Front sheet |
| 3 | Back sheet |
| 4 | Absorbent body |
| 5 | Cover sheet |
| 61-64 | Elastic members |
| 7 | Absorbent core |
| 8 | Nonwoven fabric sheet |

**Claims**

1. An absorbent article including a liquid-permeable front sheet, a liquid-impermeable back sheet, an absorbent body situated between the front sheet and the back sheet, and a nonwoven fabric sheet for the absorbent body,

   wherein the nonwoven fabric sheet includes:

   a pulp fiber layer that includes pulp and has a first surface and a second surface,
   a first surface side fiber layer situated on a first surface side of the pulp fiber layer and including mainly hydrophilic fibers with a mean fiber length of 25 mm to 64 mm, and
   a second surface side fiber layer situated on a second surface side of the pulp fiber layer and including mainly hydrophilic fibers with a mean fiber length of 25 mm to 64 mm.

2. The absorbent article according to claim 1, wherein the fiber layer of either or both the first surface side fiber layer and second surface side fiber layer further includes hydrophobic fibers in a smaller amount than the hydrophilic fibers, as the mass ratio.

3. The absorbent article according to claim 2, wherein the hydrophobic fibers include thermoplastic fibers.

4. The absorbent article according to claim 3, wherein the thermoplastic fibers are hydrophilic-treated polyester-based fibers.

5. The absorbent article according to any one of claims 1 to 4, wherein the hydrophilic fibers in the fiber layer of either or both the first surface side fiber layer and second surface side fiber layer have irregular cross-sections.

6. The absorbent article according to any one of claims 1 to 5, wherein the hydrophilic fibers in the fiber layer of either or both the first surface side fiber layer and second surface side fiber layer include cellulosic fibers.

7. The absorbent article according to any one of claims 1 to 6, wherein the pulp in the pulp fiber layer includes fluff pulp.

8. The absorbent article according to any one of claims 1 to 7, wherein the pulp in the pulp fiber layer includes pulp with a Kajaani mean fiber length of 2 to 3 mm.

9. The absorbent article according to any one of claims 1 to 8, which includes a structure in which the fibers of each of the fiber layers are tangled together by an action of a water stream.

10. The absorbent article according to any one of claims 1 to 9, wherein the fiber layer of either or both the first surface side fiber layer and second surface side fiber layer includes a carded web.

11. The absorbent article according to any one of claims 1 to 10, which includes a structure in which a portion of fibers in the fiber layer of either or both the first surface side fiber layer and second surface side fiber layer are incorporated inside the pulp fiber layer.

12. The absorbent article according to any one of claims 1 to 11, wherein a fluid absorption height of the fiber layer of either or both the first surface side fiber layer and the second surface side fiber layer is higher than the fluid absorption height of the pulp fiber layer, in a water absorption test by the Klemm method.

13. The absorbent article according to any one of claims 1 to 12, wherein the absorbent body includes an absorbent core, and the nonwoven fabric sheet is situated at least between the back sheet and the absorbent core.

14. A method for producing the nonwoven fabric sheet for the absorbent body to be used in the absorbent article according to any one of claims 1 to 13, the method including:

a step of supplying a fiber web for the second surface side fiber layer including hydrophilic fibers,
a step of supplying pulp for the pulp fiber layer including hydrophilic fibers onto the fiber web,
a step of supplying a fiber web for the first surface side fiber layer including hydrophilic fibers onto the pulp, to obtain a layered body, and
a step of high-pressure stream jet treatment from both surface sides of the layered body to tangle together at least the fibers of each of the fiber layers.

# FIG. 1

# FIG. 2

FIG. 3

FIG. 4

## INTERNATIONAL SEARCH REPORT

| International application No. |
|---|
| PCT/JP2015/052957 |

### A. CLASSIFICATION OF SUBJECT MATTER
*A61F13/49(2006.01)i, A61F13/15(2006.01)i, A61F13/53(2006.01)i, D04H1/4374 (2012.01)i, D04H1/492(2012.01)i*

According to International Patent Classification (IPC) or to both national classification and IPC

### B. FIELDS SEARCHED

Minimum documentation searched (classification system followed by classification symbols)
A61F13/00, A61F13/15-13/84, D04H1/4374, D04H1/492

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-9356 A  (Daiwabo Co., Ltd.),<br>18 January 2007 (18.01.2007),<br>paragraphs [0013] to [0038]; fig. 1<br>& JP 4721788 B2 | 14<br>1-13 |
| Y | JP 10-273884 A  (Chisso Corp.),<br>13 October 1998 (13.10.1998),<br>paragraphs [0016] to [0044], [0064] to [0065];<br>fig. 1 to 2<br>& JP 4324982 B2 | 1-13 |
| Y | JP 2012-110364 A  (Uni-Charm Corp.),<br>14 June 2012 (14.06.2012),<br>paragraphs [0016] to [0018]<br>& JP 5679777 B2            & US 2013/0245589 A1<br>& WO 2012/067216 A1      & EP 2640331 A1<br>& TW 201233377 A1        & CN 103221011 A | 12-13 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    22 April 2015 (22.04.15) | Date of mailing of the international search report<br>    12 May 2015 (12.05.15) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (July 2009)

**INTERNATIONAL SEARCH REPORT**

International application No.

PCT/JP2015/052957

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-298387 A  (Daio Paper Corp.),<br>28 October 2004 (28.10.2004),<br>paragraphs [0071] to [0072]; fig. 2<br>& JP 4297714 B2          & US 2006/0116651 A1<br>& US 7847145 B2          & WO 2004/087028 A1<br>& EP 1614408 A1          & EP 1614408 B1<br>& EP 2033608 A1          & EP 2033608 B1 | 13 |

Form PCT/ISA/210 (continuation of second sheet) (July 2009)

**EP 3 143 976 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4089053 A